(19) Europäisches Patentamt
European Patent Office
Office européen des brevets

(11) Publication number : **0 472 304 A2**

(12) # EUROPEAN PATENT APPLICATION

(21) Application number : **91306935.7**

(51) Int. Cl.$^5$ : **A61K 31/695**

(22) Date of filing : **29.07.91**

(30) Priority : **27.07.90 HU 464690**

(43) Date of publication of application :
**26.02.92 Bulletin 92/09**

(84) Designated Contracting States :
**AT BE CH DE DK ES FR GB GR IT LI LU NL SE**

(71) Applicant : **Richter Gedeon Vegyészeti Gyár R.T.**
**Gyömröi ut 19-21**
**H-1475 Budapest X (HU)**

(72) Inventor : **Farkas, Sándor**
**22, L rántffy Zsuzsanna utca**
**H-1022 Budapest (HU)**
Inventor : **Földeák, Sándor**
**5, Palánk utca**
**H-6720 Szeged (HU)**
Inventor : **Kárpáti, Egon**
**7/b, Mihályfi E. utca**
**H-1022 Budapest (HU)**
Inventor : **Hegyes, Péter**
**7/a, Vedres utca**
**H-6726 Szeged (HU)**
Inventor : **Kreidl, János**
**51, Pusztaszeri ut**
**H-1025 Budapest (HU)**
Inventor : **Szporny, Lászl**
**7, Szabolcska Mihaly ut**
**H-1111 Budapest (HU)**
Inventor : **Czibula, Lászl**
**18, Gergely utca**
**H-1103 Budapest (HU)**
Inventor : **Vassné Petofi, Szilvia**
**7/a Als kikötosor**
**H-6726 Szeged (HU)**

(74) Representative : **Pett, Christopher Phineas et al**
**Frank B. Dehn & Co. European Patent Attorneys Imperial House 15-19 Kingsway**
**London WC2B 6UZ (GB)**

(54) **Pharmaceutical compositions containing therapeutically active organosilane derivatives and process for preparing same.**

(57) The invention relates to pharmaceutical compositions containing an active ingredient at least one known organosilane derviative of formula (I),

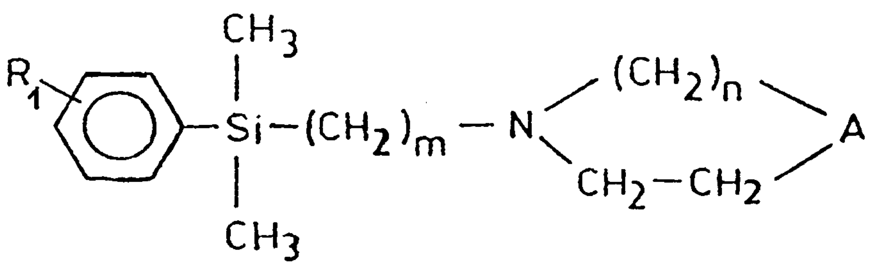

(I)

wherein

$R_1$ represents a hydrogen or halogen atom ;

A represents oxygen or $=CH_2$ or NR group, wherein R represents a hydrogen atom or a $C_{1-4}$alkyl group ;

m is 1, 2 or 3 ; and

n is 1 or 2,

or a pharmaceutically acceptable salt thereof in admixture with one or more pharmaceutically acceptable carriers, excipients and/or diluents.

The compositions of the invention possess muscle relaxant and antiparkinsonic effects, thus, they can be used for the treatment or prevention of diseases and conditions accompanied by hyperreflexia of the central nervous system and/or an increase of the muscular tone as well as nervous system injuries, degenerative nervous system disoders, sclerosis multiplex, myelopathies, rachicele, articular pains and Parkinson's disease in mammals including man.

EP 0 472 304 A2

This invention relates to the use of organosilane derivatives and salts in the treatment of reflex and muscle tone disorders and to pharmaceutical compositions containing these therapeutically active organosilane derivatives.

The invention is based on the recognition that known compounds of the formula (I),

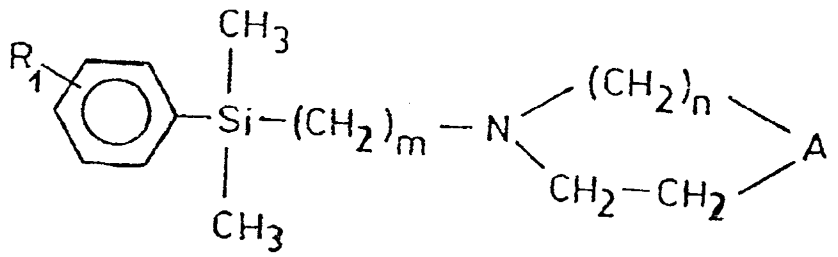

(I)

wherein

$R_1$ represents a hydrogen or halogen atom;

A represents oxygen or $=CH_2$ or -NR group, wherein R represents a hydrogen atom or a $C_{1-4}$alkyl group;

m is 1, 2 or 3; and

n is 1 or 2

and salts thereof have therapeutically useful, in particular central muscle relaxant and antiparkinsonic, effects. The invention also relates to a process of preparing the pharmaceutical compositions containing the compounds of formula (I).

The preparation and use of compounds of formula (I) has been published in a high number of papers and patent specifications.

A. Tzschach et al. describe N-heterocyclic compounds containing silicone as important intermediates of chemical reactions 17 (1984)].[J. of Organometallic Chem. 266, 17 (1984)].

The preparation of N-heterocyclic compounds is described in the European patent specification No. 0,224,024. These compounds are used for the preparation of pesticides.

The preparation and fungicidal action of organosilane derivatives containing nitrogen is described in a Russian publication (Latv. PSR Zinat. Akad. Vestis, Kim. Ser. 1978, 343).

It has surprisingly been found during our investigations that the organosilane compounds of formula (I) and salts thereof can also be used in human therapy, in particular for the treatment of disorders and conditions characterized by an increase in the reflexes (hyper-reflexia) or an increase in the muscular tone (hypertonicity; spasticity or rigidity).

The disorders mentioned above affect a significant part of the population. For the treatment and alleviation of such conditions central muscle relaxant drugs, e.g. tolperisone are used, however several muscle relaxants including tolperisone are not satisfactorily effective and have a short duration of effect.

Thus, one aspect of this invention relates to a pharmaceutical composition comprising as active ingredient at least one organosilane derivative of formula (I)

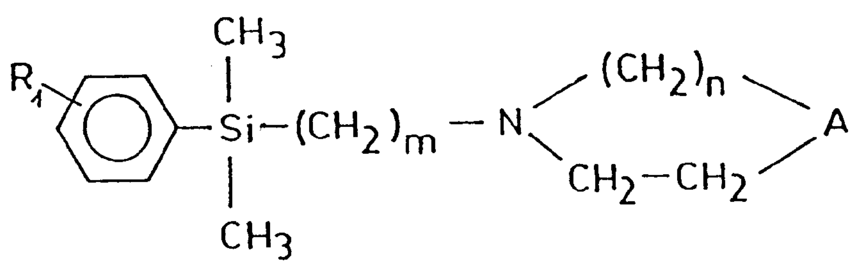

(I)

wherein $R_1$, A, m and n are as hereinbefore defined or a pharmaceutically acceptable salt thereof in admixture with one or more pharmaceutically acceptable carriers, excipients and/or diluents.

On the basis of pharmacological results, we have found that many members of the compounds of formula (I) (which term is used hereafter to include salts of the compounds of formula (I)) possess excellent central muscle relaxant and antiparkinsonic effects exceeding in several respects the commercially available muscle relaxants. These compounds as well as the structurally related tolperisone and eperisone were investigated by using various methods indicating (demonstrating) the central muscle relaxant effects, and their comparative

effects.

The compounds particularly investigated are numbered in the Tables summarizing the pharmacological results as follows:

1 N-[(dimethyl-phenylsilyl)methyl]piperidine fumarate
3 N-[(dimethyl-phenylsilyl)methyl]pyrrolidine hydrochloride
4 N-{[dimethyl-(4-fluorophenyl)silyl]methyl}piperidine fumarate
5 N-{[dimethyl-(4-fluorophenyl)silyl]methyl}piperidine hydrochloride
6 N-{[dimethyl-(4-fluorophenyl)silyl]methyl}pyrrolidine hydrochloride.

The methods and results of pharmacological investigations carried out on the compounds are discussed hereinafter.

Method 1: Test on the GYKI-20039-induced tremor

Groups consisting of 10 to 20 male CFLP mice each weighing 19 to 21 g were intraperitoneally (i.p.) treated with 10 mg/kg of GYKI-20039 (test substance). Similarly to the structurally related compound of code number LON-954, GYKI-20039 induces a tremor on mice which is considered as the experimental model of the Parkinson's disease [Coward et al., Psychopharmacology 52, 165 (1977); Andrási et al. IX Internat. Congress of Neuropathology, I-98, 209, Vienna 1982]. The tremor induced by GYKI-20039 reaches a maximum strength 4 to 8 minutes following administration of the test substance, then it continuously diminishes and is finished within about 1 hour. According to our experiences obtained on ten known central muscle relaxants, this tremor can be inhibited by central muscle relaxants in a dose-dependent manner and is useful for the assay (evaluation) of the central muscle relaxant effects. After administration of the test substance, the mice were suspended on an isometric dynamometer in a light plastic box and the strength of the tremor was measured and quantified by electronic integration. The tremor strength of the mice was averaged in the 4th-8th minutes after treatment with the test substance.

The animals were pretreated with various doses of the muscle relaxants to be tested before the treatment with the test substance. The time of pretreatment was 0 mins in the intravenous examinations whereas a pretreatment time of 10 minutes was used in oral experiments. The $ED_{50}$ values of the substances to be tested, i.e. the dose causing an inhibition of 50%, were determined from the logarithmic dose/response curves by linear regression. The arithmetic mean of the integrated activity values of the group treated with the test substance or the untreated control group, respectively was considered to be the inhibition of 50%.

Method 2: Straub's tail test

The method of Novack [Drug Development Res. 2, 383 (1982)] was used with a slight modification. Groups consisting of 10 male OF-1 mice each weighing 19 to 21 g were intraperitoneally treated with 60 mg/kg of morphine hydrochloride. The formation of Straub's tail was judged 15 minutes after the morphine treatment. This was considered to be positive when the animal kept his tail permanently upwards at an angle being steeper than 45°. A pretreatment was carried out with various doses of the compound to be tested 20 minutes before the time of reading. The $ED_{50}$ value, i.e. the dose inhibiting the positive response in 50% of the animals, was calculated by using the method of Litchfield and Wilcoxon.

(The above author found a good relation between the activity shown in the above test and the clinical effectivity of central muscle relaxants.)

Method 3: Investigation of the flexor reflex on anaesthetized cats

Chloralose-anaesthetized cats of both sexes weighing 2.2-5.4 kg each were used in these experiments. The flexor reflex of the animals was studied by using the quantitative electromyographic method of Farkas et al [Pharmacol. Res. Comm. 20, Suppl. 1, 141 (1988)].

The effects of 10 mg/kg doses of the various compounds were investigated for evaluating the strength of effectivity. The results summarized in Table 1 are the values calculated from the average results of 4 to 8 experiments. The duration of effect was characterized by the time passing from the administration of this dose until the return to 75% of the control value ($T_{75\%}$).

On the basis of the pharmacological investigations the compounds according to the invention possess central muscle relaxant effects. The pharmacological activities of the compounds showing the most prominent effects together with the activity of tolperisone used as reference drug are shown in Table 1. Each of the compounds appearing in Table 1 resulted in an inhibition of 50 to 80 % of the flexor reflex in the dose used.

Table 1

| Mice | | | Flexor reflex on cats 10 mg/kg i.v. |
|---|---|---|---|
| Compound | GYKI-20039 $ED_{50}$ (mg/kg) i.v. | Straub's tail $ED_{50}$ (mg/kg) i.p. | Duration of effect $T_{75\%}$ (min) |
| Tolperisone | 10.2 | 63.0 | 50 |
| 1 | 4.6 | 30.7 | - * |
| 3 | 4.2 | 37.7 | 50** |
| 4 | 7.9 | 19.1 | 180 |
| 5 | 3.2 | 17.8 | > 180 |
| 6 | 5.4 | 26.9 | > 180 |

Notes:

* : it was not investigated

** : the effect of 5 mg/kg was investigated

The numbers 1, 3, 4, 5 and 6 are the numbers of the Examples hereinafter describing the preparation of the compounds.

It is obvious from the results shown in Table 1 that, both on intravenous or intraperitoneal administration, the effectiveness of the compounds according to the invention is more than 2 to 3 times higher than that of tolperisone. The compounds according to the invention inhibited the flexor reflex on cats similarly to tolperisone, but unexpectedly, the duration of effect of some representatives of the compounds of formula (I) was more than 3-fold of that of tolperisone used as reference substance. The comparison of compound No. 4 with compound No. 5 shows that both the hydrochloride and the fumarate of these compounds are active and the effectivity of the hydrochloride salts usually surpasses that of the fumarates.

It can clearly be seen from the pharmacological results that the compounds according to the invention and their pharmaceutically acceptable salts possess outstanding central muscle relaxant properties in comparison to known compounds and in addition, they effectively inhibit the experimentally induced tremor considered to be the model of the Parkinson's disease. Thus, being central muscle relaxants, they may be useful for the treatment of conditions accompanied by spastic or rigid hypertonicity, stroke, nervous system injuries, degenerative nervous dystem disorders, sclerosis multiplex, myelopathies, rachicele, articular pains, Parkinson's disease and syndrome as well as similar conditions on mammals (including man) both in the oral or parenteral route.

It can be expected that, depending on the body-weight, age, sex and route of the administration, the compounds acdording to the invention exert their therapeutic effects in a dose range of 5 to 500 mg/day.

In one embodiment of this invention, the pharmaceutical compositions of the invention have muscle

relaxant and optionally antiparkinsonic effects. They also appear useful for the prevention and treament of disorders accompanied by hyperreflexia of the central nervous system and/or an increase in the muscular tone (hypertonicity).

According to another aspect of the invention, there is provided a process for the prepartion of pharmaceutical compositions containing as active ingredient at least one organosilane derivatives of formula (I), or salts thereof which comprises admixing the known active ingredient(s) with one or more pharmaceutically acceptable carriers, excipients, and/or diluents.

The compounds of formula (I) may be formulated into pharmaceutical compositions by mixing them with nontoxic inert solid or liquid carriers and/or additives commonly used which are useful for parenteral or enteral administration. Suitable carriers are e.g. water, gelatine, lactose, starch, pectin, magnesium stearate, stearic acid, talc, vegetable oils such as peanut oil or olive oil and the like. The active ingredients may also be formulated into other usual pharmaceutical compositions, particularly to solid forms, e.g. rounded or angled tablets, dragées, capsules such as gelatine capsules, pills and the like.

The amount of the solid carrier may be varied within a wide range, preferably between about 1 mg and 500 mg. If desired, the compositions may contain the usual pharmaceutical additives, e.g. preservatives, stabilizers, wetting agents (surfactants), emulsifying agents and the like. These compositions, e.g. solid compositions, may be prepared by using known methods, e.g. sieving, mixing, granulating and compressing the ingredients. The compositions may be subjected to other usual procedures (operations) of the pharmaceutical technology, e.g. to sterilization.

A preferred variation of tablet formulation contains an excipient, disintegrating and sliding agent in addition to the active ingredient.

According to a preferred method of suspension formulation the active ingredient is milled to the desired particle size and then uniformly dispersed in a syrup containing the flavoring agent, such as raspberry or strawberry flavour, colouring agent, food dye, viscosity-increasing agent, e.g. Carbopol, surfactant, e.g. Tween as well as a preservative, e.g. sodium benzoate.

The preparation of pharmaceutical compositions containing the compound of formula (I) is illustrated in the following Example without limiting the scope of our invention to this formulation.

Formulation Example 1

Preparation of tablets

| Ingredients of one tablet: | mg |
|---|---|
| Active ingredient | 50 |
| Gelatine | 3 |
| Magnesium stearate | 2 |
| Talc | 5 |
| Potato starch | 45 |
| Lactose | 95 |
| Total weight | 200 |

The active ingredient is mixed with the lactose and three quarters of the potato starch, then the homogeneous mixture obtained is kneaded together with the aqueous solution of gelatine and granulated. After drying the wet granulate at a temperature between 30°C and 50°C, talc and the remaining quarter of potato starch as well as magnesium stearate are added to the dried granulate. The mixture obtained is compressed into tablets. Optionally (if desired) the tablets are provided with a groove for facilitating their administration.

A yet further aspect of this invention is the use of a compound of formula (I) as hereinbefore defined or a pharmaceutically acceptable salt thereof in the manufacture of a medicament having muscle relaxant and optionally antiparkinsonic effects. A further embodiment of this invention is the use of a compound of formula (I) as hereinbefore defined or a pharmaceutically acceptable salt thereof in the manufacture of a medicament for the prevention and treatment of disorders accompanied by hyperreflexia of the central nervous system and/or an increase in the musclar tone (hypertonicity).

The preferred variants of the preparation of active ingredients of the pharmaceutical compositions according to the invention are illustrated in the following Examples.

Example 1

N-[(dimethyl-phenylsilyl)methyl]piperidine fumarate

9.2 g of chloromethyl-dimethyl-phenylsilane are reacted with 10.7 g of piperidine in 50 ml of xylene by refluxing for 4 hours. After cooling down, the solution is washed 5 times with a total of 250 ml of water. The organic layer is dried over a drying agent, filtered and evaporated to solvent-free. The pale yellow oily residue is dissolved in 40 ml of ether and 6 g of fumaric acid are added. After boiling the mixture, the crystalline precipitate is filtered at 0 °C to give the title salt product in a yield of 14.8 g (85 %), m.p.: 171-175 °C.

Example 2

N-[(dimethyl-phenylsilyl)methyl]pyrrolidine fumarate

Example 1 is followed, except that 8.9 g of pyrrolidine are used as starting substance instead of 10.7 g of piperidine. The title product is obtained in a yield of 14.4 g (86 %), m.p.: 113 °C.

Example 3

N-[(dimethyl-phenylsilyl)methyl]pyrrolidine hydrochloride

Example 1 is followed, except that the ethereal solution of the base is acidified to a pH value of 4 by adding hydrogen chloride in isopropanol solution. The title hydrochloride salt is obtained in a yield of 10 g (78.5%), m.p.: 142.5-143.5 °C.

Example 4

N-{[dimethyl-(4-fluorophenyl)silyl)methyl}piperidine fumarate

A solution containing 10.1 g of chloromethyl-dimethyl-(4-fluorophenyl)silane and 10.7 g of piperidine in 50 ml of toluene is refluxed for 12 hours, then washed 5 times with a total of 250 ml of water. The organic phase is dried, filtered an evaporated to solvent-free. The pale yellow oily residue is dissolved in 40 ml of ether and 6 g of fumaric acid are added. After boiling the mixture the crystalline precipitate is filtered at 0 °C to give the title salt product in a yield of 15.6 g (86%), m.p.: 141 - 144 °C.

Example 5

N-{[dimethyl-(4-fluorophenyl)silyl)methyl}piperidine hydrochloride

Example 1 is followed, except that the ethereal solution of the base is acidified to a pH value of 4 by adding hydrogen chloride in isopropanol solution. The title salt product is obtained in a yield of 11.5 g (80%), m.p.: 139-140 °C.

Example 6

N-{[dimethyl-(4-fluorophenyl)silyl]methyl}pyrrolidine hydrochloride

Example 5 is followed, except that 8.9 g of pyrrolidine are used as starting substance instead of 10.7 g of piperidine. The title hydrochloride salt is obtained in a yield of 10.8 g (79%), m.p.: 145 °C.

Example 7

N-{[dimethyl-(4-fluorophenyl)silyl)propyl}morpholine hydrochloride

A solution containing 11.5 g of chloropropyl-dimethyl-(4-fluorophenyl)silane and 11 g of morpholine in 50 ml of benzene is refluxed for 16 hours, then cooled down and washed 5 times with a total of 250 ml of water. The organic phase is dried over a drying agent, filtered and evaporated to solvent-free. The slightly yellow oily residue is dissolved in 5 ml of ethyl acetate and acidified to pH 4 by adding hydrogen chloride in isopropanol

solution. The crystalline precipitate is filtered at 0 °C to obtain the title hydrochloride in a yield of 13.3 g (84 %), m.p.: 177-178 °C.

Example 8

$N^1$-butyl-$N^4$-{[dimethyl-(4-fluorophenyl)silyl]propyl}piperazine hydrochloride

Example 7 is followed, except that 17.8 g of N-butylpiperazine are used as starting substance instead of 11 g of morpholine and the salt-forming mixture is acidified to pH 1 (instead of pH 4). The title dihydrochloride salt is obtained in a yield of 14.2 g (72 %), m.p.: 206.5-207.5 °C.

## Claims

1. A pharmaceutical composition comprising at least one organosilane derivative of formula (I),

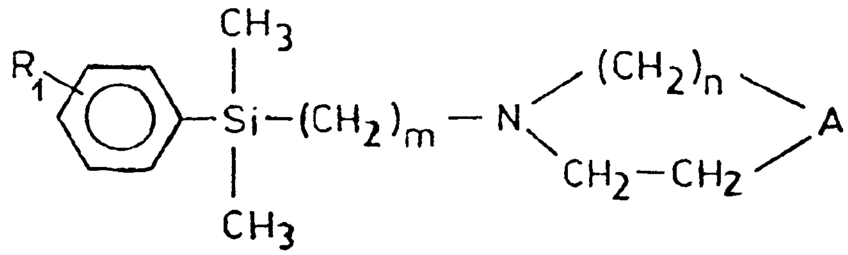

(I)

wherein

$R_1$ represents a hydrogen or halogen atom;

A represents oxygen or $=CH_2$ or NR group, wherein R represents a hydrogen atom or a $C_{1-4}$alkyl group;

m is 1, 2 or 3; and

n is 1 or 2,

or a pharmaceutically acceptable salt thereof in admixture with one or more pharmaceutically acceptable carriers, excipients and/or diluents.

2. A pharmaceutical composition as defined in Claim 1 having muscle relaxant and optionally antiparkinsonic effects.

3. A pharmaceutical composition as defined in Claim 1 for the prevention and treatment of disorders accompanied by hyperreflexia of the central nervous system and/or an increase in the muscular tone (hypertonicity).

4. A process for the preparation of a pharmaceutical composition as defined in any one of Claims 1-3 which comprises admixing at least one organosilane derivative of formula (I) or a pharmaceutically acceptable salt thereof with one or more pharmaceutically acceptable carriers, excipients and/or diluents.

5. The use of a compound of formula (I) as defined in Claim 1 or a pharmaceutically acceptable salt thereof in the manufacture of a medicament having muscle relaxant and optionally antiparkinsonic effects.

6. The use of compound of formula (I) as defined in Claim 1 or a pharmaceutically acceptable salt thereof in the manufacture of a medicament for the prevention and treatment of disorders accompanied by hyperreflexia of the central nervous system and/or an increase in the muscular tone (hypertonicity).

7. The use of a compound of formula (I) as defined in claim 1 for the prevention and treatment of disorders accompanied by hyperreflexia of the central nervous system and/or an increase in the muscular tone (hypertonicity)